# EUROPEAN PATENT SPECIFICATION

(11) **EP 2 311 521 B1**
(45) Date of publication and mention of the grant of the patent: **02.03.2016**
(21) Application number: 10187877.5
(22) Date of filing: 18.10.2010
(51) Int. Cl.: A61N 1/14, A61N 1/16, H01Q 17/00

(54) **Device for attenuating electromagnetic radiation present in a space**
Vorrichtung zur Abschwächung elektromagnetischer Strahlung
Dispositif pour atténuer le rayonnement électromagnétique présent dans un espace

(30) Priority: 16.10.2009 NL 2003651
(43) Date of publication of application: 20.04.2011
(73) Proprietor: Tol, Joseph Maria, 1132BB Volendam (NL)
(72) Inventor: Tol, Joseph Maria, 1132BB Volendam (NL)
(74) Representative: Mink-Lindenburg, Charlotte Hildegard

(56) References cited:
- EP-A1- 0 131 636
- WO-A1-98/06453
- WO-A1-2007/023376
- DE-A1- 3 304 155
- DE-A1- 3 900 652
- DE-A1- 10 315 992
- DE-U1- 9 102 248
- US-A1- 2004 041 106
- US-A1- 2009 149 933

## Description

The invention relates to a device for attenuating electromagnetic radiation present in a space, comprising an antenna for collecting and discharging the electromagnetic radiation provided with one or more coils.

There is increasing concern about the possible harmful effects which can occur through exposure to electromagnetic radiation, particularly in the radio frequency range, such as GSM and UMTS signals. Recent studies have shown that exposure to electromagnetic fields can cause a pathogenic effect in humans and animals. Reference is made here to a study commissioned by the European Parliament into "the physiological and environmental effects of non-ionizing electromagnetic radiation", PE no. 297.574, March 2001. It is known of electromagnetic rays that they can be reflected by inside and outside walls and that they reach their maximum intensity at the locations where the waves come into contact with a surface, such as for instance an outside or inside wall (source: Bundesamt für Kommunikation BAKOM, Switzerland). It is also known that natural fields on the earth's surface can locally change character, whereby they become a risk factor in respect of the health of people residing at this location, certainly when combined with electromagnetic fields and resonances thereof as stated above. Reference is made here to the publication Risikofaktor Standort: "Rutengängerzone und Mensch. Wissenschaftliche Untersuchung zum Problem der Standorteinflüsse auf den Menschen", ISBN 978-3-85076-276-2, by Dr. Otto Bergsman.

It is assumed that the normal regulating processes of a living organism act with a small control energy such that these processes function differently in the case of a minimal variation in the control energy. This energy change can be caused by electromagnetic radiation. The invention has for its object to provide a device for attenuating the electromagnetic radiation present in a space in order to thus reduce the risk of pathogenic effects. Such a device is known in the field. The known device is described in the American patent US 6,011,523. This patent describes a device for transforming the frequency of harmful electromagnetic radiation to a frequency occurring naturally close to earth. The device is provided for this purpose with a number of coils arranged in parallel and special powder arranged around the coils.

The present device according to the invention does not however attenuate the harmful electromagnetic radiation by transformation but by discharge thereof to the earth. The device stated in the preamble has for this purpose the feature that the antenna is substantially rod-like, that the coils are arranged round the antenna such that the coils are coupled to the antenna and that the device can be connected to earth for the purpose of discharging the electromagnetic radiation to earth. The antenna makes use of the phenomenon that, when the electromagnetic waves are received in conductors, a resonance is created, the energy of which is discharged to earth. The present invention is also able to collect and/or discharge the resonances of the electromagnetic radiation present in the air.

In the device according to the invention in accordance with the preamble of claim 1 the windings of each coil are provided with a first outer end and a second outer end which extend substantially perpendicularly of the antenna. The technical effect hereof is to collect and conduct the electromagnetic resonances in the space.

The device according to the preamble of claim 1 is known from the international patent application WO2007/023376. WO 98/06453 discloses a microwave absorber with two crossed windings, inside which is a "V" bent conductor. The known device is however only suitable for collecting earth radiation and discharge thereof to earth. It is the object of the invention to collect not only earth radiation but also electromagnetic radiation caused by humans, and to further increase discharge thereof to earth.

For this purpose a metal wire is arranged on the antenna at the position of the upper side of each coil. In this way the first outer end and the second outer end of the coil are supported. This has the surprising effect that more electromagnetic radiation is collected and discharged to earth.

The metal wire is preferably connected to the antenna by means of one or more windings round the antenna.

The metal wire is preferably wound around the first and second outer ends.

Both the above stated measures enhance the surprising effect.

In a preferred embodiment of the device according to the invention the device comprises an upper, middle and lower coil as seen in longitudinal direction of the rod. This has the surprising effect that the collection of electromagnetic radiation and discharge thereof to earth is further increased.

In a practical preferred embodiment of the device according to the invention the ratio of the number of windings of the upper, middle and lower coil is 9:5:8. The technical effect hereof is that resonances are collected at different specific frequencies forming a potential risk factor. In a further practical preferred embodiment of the device according to the invention the antenna is provided with a housing substantially comprising the antenna, wherein the housing is provided with a cover of successive layers of electrically and non-electrically conductive material. The housing is preferably substantially filled with a non-conductive material, such as resin, which is provided with minerals, carbon, fibre and silver foil. The technical effect of these additions is to enhance the ability to collect the radiation.

The invention also relates to a method for placing the device in the space. The method has the feature here that the device is placed on a line along the bisecting angle of two adjacent walls, wherein the first and the second outer end of the winding of each coil point in the direction of the angle between the two adjacent walls. The device according to the invention is hereby placed at the position where the resonances in a space are maximal.

The coils are preferably tuned to specific frequencies related to significant areas of risk, such as the 900 and 1800 MHZ frequencies of mobile telephony, the 2.1 GHz of the UMTS network and the 380/395 MHZ of the C2000 network. More specifically the device is extremely suitable for collecting and discharging electromagnetic radiation around 100, 360, 390, 950,1800 and 2100 MHZ.

The device according to the invention and the method according to the invention will now be further elucidated with reference to the following figures, wherein
Figure 1 shows a side view and a cross-section of the device according to the invention;
Figure 2 shows a cross-section of the device according to the invention;
Figure 3 shows a first arrangement of the device according to the invention following application of the method according to the invention;
Figure 4 shows a second arrangement of the device according to the invention following application of the method according to the invention;
Figures 5 and 6 show the measurement results from a test into the operation of the device according to the invention.

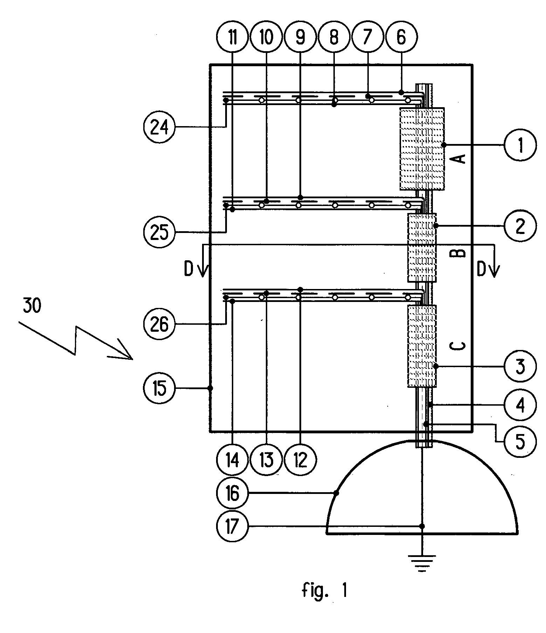

Figure 1 shows a side view of device 30 according to the invention. Device 30 comprises an antenna 5 manufactured from an aluminium rod on which a nickel cover is arranged in the longitudinal direction. Coils A, B and C are mounted on this rod 5. Each coil has two copper wires 6, 8, 9, 11, 12, 14 twisted to the left and lying (perpendicularly) on coils A, B and C. Per coil a third nickel wire 7, 10, 13 and a wire of silk (not shown) are also brought together with the two copper wires 6, 8, 9, 11, 12, 14 twisted to the left. This further reinforces the conductive action. Coil A forms the upper part of antenna 5. As seen from above, the coil direction of coils A, B and C, beginning with the first layer, is clockwise.

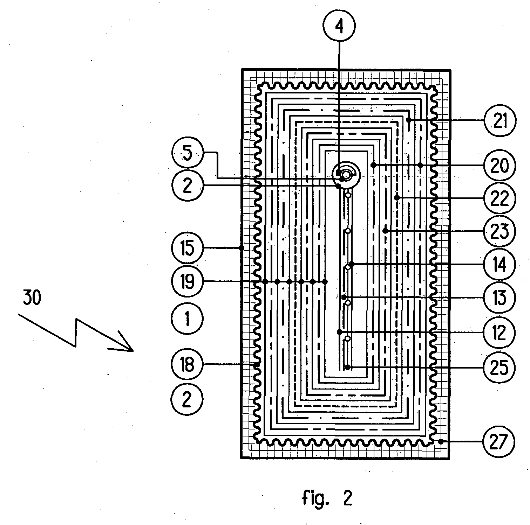

Figure 2 also shows a cross-section of device 30 according to the invention along the line DD, i.e. through coil B. A cover of different electrically conductive and non-electrically conductive materials is arranged around antenna 5. In a preferred embodiment of the invention this cover consists of successive layers of transparent foil 19, silver foil 20, transparent foil 19, carbon 21, transparent foil 19, steel wool 22, transparent foil 19, fibre 23, transparent foil 19, silver foil 20 and transparent foil 19. This entity is enclosed by a fabric cover 18 and silver foil 27. Silver foil 27 is attached above the fabric cover 18. The whole is placed in an aluminium housing 15. The inside of aluminium housing 15 is filled with a resin. Antenna 5 is attached to a chrome steel (or aluminium) half-sphere 16 and connected to earth. The inner side of half-sphere 16 is covered with a carbon layer. A layer of resin is arranged in the top of the half-sphere.

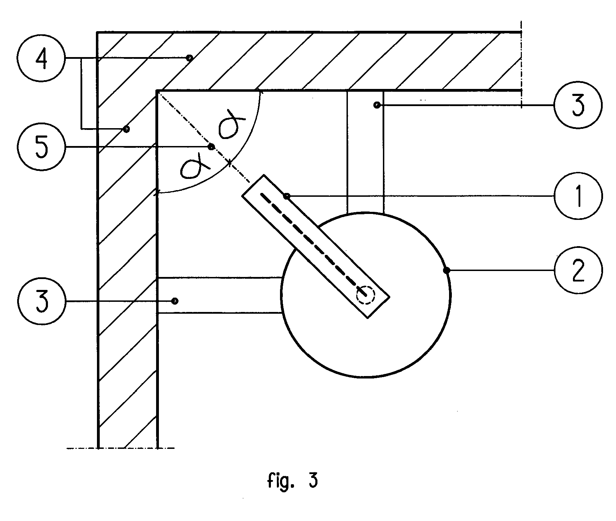

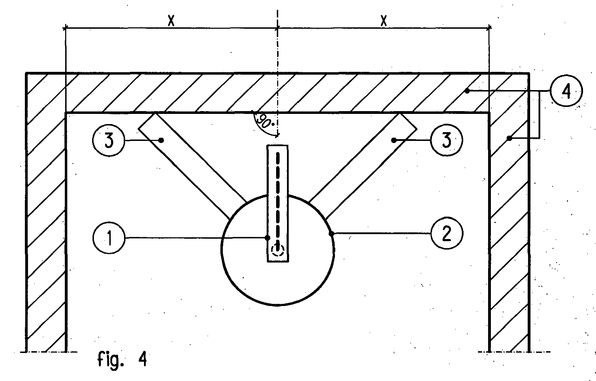

Figures 3 and 4 show an arrangement of device 30 according to the invention following application of the method according to the invention. For a correct operation of device 30 it is necessary that device 30 is placed in a bisected angle relative to antenna 5 in a space, or is placed in the middle against the wall 4 of a space. Spacer 3 ensures that this position is easy to find. Correct operation can also be achieved by placing device 30 in the middle between two opposite walls.

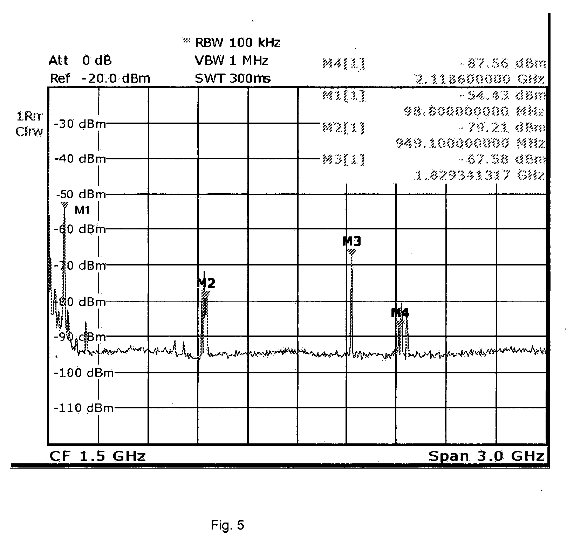

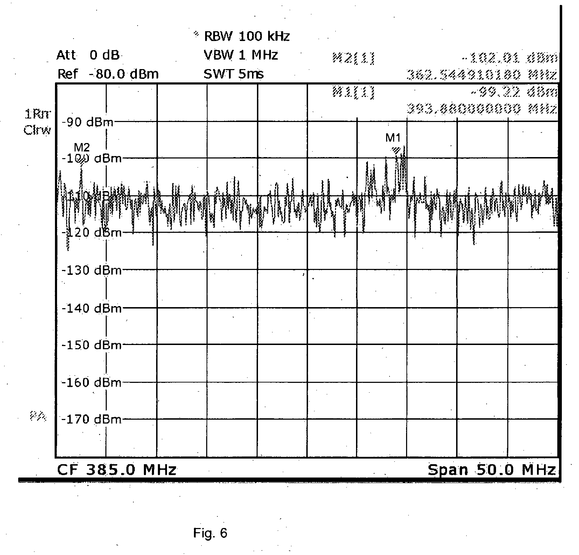

Figures 5 and 6 show the measurement results from a test into the operation of device 30 according to the invention. Device 30 according to the invention has been tested extensively with an ESCI EMI Test Receiver of the company Rohde & Schwartz. This apparatus measures electromagnetic emissions and complies with all applicable standards. The frequency range that was test measured runs from 9. kHz - 3 GHz. The results show that the antenna is able to receive the intended frequencies of the electromagnetic radiation.

Figure 5 shows the result of the measurement of the ability of the antenna to collect determined frequencies within the frequency range of 9KHz-3GHz. The x-axis indicates here the frequency in Hz and the y-axis the strength of the signal received by the antenna in dBm. Note: dBm is the designation for converting the relative value of dB (decibel) to an absolute value with a reference point: 0 dBm = 1 mW at 600 ohm = 0.775 V. It can be inferred from the measurement that the intended frequencies linked to specific areas of risk are collected more strongly than the other frequencies. The values M1-M4 are the measured maximums here. These correspond to the frequencies used by the GSM and UMTS network and the wireless DECT system.

Figure 6 shows the result of the measurement of the ability of the antenna to collect determined frequencies within the frequency range of 360 - 410 MHZ. The x-axis shows here the frequency in Hz and the y-axis the strength of the signal received by the antenna in dBm. It can be inferred from the measurement that the intended frequencies linked to specific areas of risk are collected more strongly than the other frequencies. The values M1[1] and M2[1] are the measured maximums here. These correspond to the frequencies used by the C2000 network.

## Claims

1. Device (30) for attenuating electromagnetic radiation present in a space, comprising an antenna (5) for collecting the electromagnetic radiation provided with one or more coils (A,B,C), wherein the antenna is substantially rod-like, the coils are arranged round the antenna such that the coils are coupled to the antenna, the device can be connected to earth for the purpose of discharging the electromagnetic radiation to earth and the winding of each coil is provided with a first outer end wire and a second outer end wire (6, 8, 9, 11, 12, 14) **characterized in that** the first outer end wire and second outer end wire extend substantially perpendicularly of the antenna, the first outer end wire and second outer end have free ends and that a metal wire (7, 10, 13) is arranged on the antenna at the position of the upper side of each coil for the purpose of supporting the first and second outer end wires.

2. Device as claimed in claim 1, **characterized in that** the metal wire is connected, to the antenna by means of one or more windings round the antenna.

3. Device as claimed in claim 1 or 2, **characterized in that** the metal wire is wound at least partially round the first and second outer end wires.

4. Device as claimed in claim 1, 2 or 3, **characterized in that** the device comprises an upper, middle and lower coil as seen in longitudinal direction of the rod.

5. Device as claimed in claim 4, **characterized in that** the ratio of the number of windings of the upper, middle and lower coil is 9:5:8.

6. Device as claimed in claims 1 to 5, **characterized in that** the antenna is provided with a housing substantially comprising the antenna, wherein the housing is provided with a cover of successive layers of electrically and non-electrically conductive material.

7. Device as claimed in claim 6, **characterized in that** the housing is substantially filled with a non-conductive material, such as resin, which is provided with minerals, carbon, fibre and silver foil.

8. Method for placing the device as claimed in claims 1 to 7 in the space, **characterized in that** the device is placed on a line along the bisecting angle of two adjacent walls, wherein the first and the second outer end of the winding of each coil point in the direction of the angle between the two adjacent walls.

## Patentansprüche

1. Vorrichtung (30) zum Abmildern von in einem Raum vorhandener elektromagnetischer Strahlung, umfassend eine Antenne (5) zum Sammeln der elektromagnetischen Strahlung, die mit einer oder mehreren Spulen (A, B, C) versehen ist, wobei die Antenne im Wesentlichen stabförmig ist und die Spulen um die Antenne herum angeordnet sind, so dass die Spulen mit der Antenne verbunden sind, wobei die Vorrichtung zum Ableiten der elektromagnetischen Strahlung mit der Erde verbunden werden kann und die Windung jeder Spule mit einem ersten äußeren Endkabel und einem zweiten Endkabel (6, 8, 9, 11, 12, 14) versehen ist, **dadurch gekennzeichnet, dass** das erste äußere Endkabel und das zweite äußere Endkabel sich im Wesentlichen senkrecht von der Antenne weg erstrecken, wobei das erste äußere Endkabel und das zweite äußere Endkabel freie Enden aufweisen und wobei ein Metallkabel (7, 10, 13) an der Antenne in der Position der Oberseite jeder der Spulen angeordnet ist, um das erste und das zweite äußere Endkabel zu unterstützen.

2. Vorrichtung gemäß Anspruch 1, **dadurch gekennzeichnet, dass** das Metallkabel mittels einer oder mehrerer Windungen rund um die Antenne mit der Antenne verbunden ist.

3. Vorrichtung gemäß Anspruch 1 oder 2, **dadurch gekennzeichnet, dass** das Metallkabel zumindest teilweise um das erste und das zweite äußere Endkabel herumgewunden ist.

4. Vorrichtung gemäß Anspruch 1, 2 oder 3, **dadurch gekennzeichnet, dass** die Vorrichtung in Längsrichtung des Stabes betrachtet eine obere, mittlere und untere Spule umfasst.

5. Vorrichtung gemäß Anspruch 4, **dadurch gekennzeichnet, dass** das Verhältnis der Windungen der oberen, mittleren und unteren Spule 9:5:8 ist.

6. Vorrichtung gemäß den Ansprüchen 1 bis 5, **dadurch gekennzeichnet, dass** die Antenne mit einem Gehäuse versehen ist, das im Wesentlichen die Antenne umfasst, wobei das Gehäuse mit einem Deckel aus aufeinanderfolgenden Schichten elektrisch leitenden und elektrisch isolierenden Materials versehen ist.

7. Vorrichtung gemäß Anspruch 6, **dadurch gekennzeichnet, dass** das Gehäuse im Wesentlichen mit einem nicht leitenden Material, wie Harz, gefüllt ist, das mit Mineralien, Kohlenstoff, Fiberglas und Silberfolie versehen ist.

8. Verfahren zum Anordnen der Vorrichtung gemäß den Ansprüchen 1 bis 7 in den Raum, **dadurch gekennzeichnet, dass** die Vorrichtung auf einer Linie entlang der Winkelhalbierenden zwischen zwei benachbarten Wänden angeordnet ist, wobei das erste und das zweite äußere Ende der Windung jeder Spule in die Richtung der Ecke zwischen den zwei benachbarten Wänden zeigt.

## Revendications

1. Dispositif (30) pour atténuer le rayonnement électromagnétique présent dans un espace, comprenant une antenne (5) pour collecter le rayonnement électromagnétique et munie d'une ou plusieurs bobines (A, B, C), dans lequel l'antenne est sensiblement en forme de barre, les bobines sont disposées autour de l'antenne de telle sorte que les bobines sont couplées à l'antenne, le dispositif peut être relié à la terre dans le but de décharger le rayonnement électromagnétique à la terre et l'enroulement de chaque bobine est munie d'un premier fil d'extrémité extérieur et d'un deuxième fil d'extrémité extérieur (6, 8, 9, 11, 12, 14), **caractérisé en ce que** le premier fil d'extrémité extérieur et le deuxième fil d'extrémité extérieur s'étendent sensiblement perpendiculairement à l'antenne, le premier fil d'extrémité extérieur et le deuxième fil d'extrémité extérieur ont des extrémités libres et **en ce qu'**un fil métallique (7, 10, 13) est disposé sur l'antenne à l'endroit de la face supérieure de chaque bobine dans le but de soutenir les premier et deuxième fils d'extrémité extérieurs.

2. Dispositif selon la revendication 1, **caractérisé en ce que** le fil métallique est connecté à l'antenne par l'intermédiaire d'un ou plusieurs enroulements autour de l'antenne.

3. Dispositif selon la revendication 1 ou 2, **caractérisé en ce que** le fil métallique est enroulé au moins partiellement autour des premier et deuxième fils d'extrémité extérieurs.

4. Dispositif selon la revendication 1, 2 ou 3, **caractérisé en ce que** le dispositif comprend une bobine supérieure, intermédiaire et inférieure, considéré dans la direction longitudinale de la barre.

5. Dispositif selon la revendication 4, **caractérisé en ce que** le rapport entre le nombre de spires de la bobine supérieure, intermédiaire et inférieure est de 9:5:8.

6. Dispositif selon les revendications 1 à 5, **caractérisé en ce que** l'antenne est munie d'un boîtier comprenant essentiellement l'antenne, le boîtier étant pourvu d'un couvercle composé d'une succession de couches de matériaux conducteurs d'électricité et non conducteurs d'électricité.

7. Dispositif selon la revendication 6, **caractérisé en ce que** le boîtier est rempli sensiblement d'un matériau non conducteur, tel que de la résine, qui est doté de minéraux, de carbone, de fibres et de feuilles d'argent.

8. Procédé pour placer le dispositif selon les revendications 1 à 7 dans l'espace, **caractérisé en ce que** le dispositif est placé sur une ligne le long de la bissectrice de deux parois adjacentes, la première et la deuxième extrémité extérieure de l'enroulement de chaque bobine pointant dans la direction de l'angle situé entre les deux parois adjacentes.
